# EUROPEAN PATENT APPLICATION

(11) **EP 2 975 047 A1**
(43) Date of publication of application: **20.01.2016**
(21) Application number: 14002513.1
(22) Date of filing: 18.07.2014
(51) Int. Cl.: C07K 14/47

(54) **Wnt7a polypeptides and their use**

(71) Applicant: Université de Lausanne, 1011 Lausanne (CH)
(72) Inventor: Katanaev, Vladimir, 1510 Moudo (CH); Koval, Alexey, 2000 Neuchâtel (CH); Blagodatski, Atem, B -24-213 (RU)
(74) Representative: Helbig, Christian

(57) **Abstract**

The invention relates to polypeptide fragments of a Wnt7a polypeptide and their use in cancer prevention and treatment.

## Description

The invention relates to novel Wnt7a polypeptides, compositions comprising same and their use in cancer treatment.

### Background of the Invention

The triple-negative breast cancer (TNBC) is known to be the most malignant breast cancer form, due to lack of HER2, ER and PR receptors, and is therefore incurable through common hormone- or monoclonal-based therapies. It is known that aberrant expression of the Wnt signaling pathway components is a common cause of breast tissue malignization.

Multiple research data confirm the active participation of the canonical Wnt pathway in the formation and proliferation of breast cancer, including the most aggressive and poorly curable triple-negative breast cancer (TNBC). Wnt ligands are a higly diverse family of proteins: whereas some of them are potent activators of the canonical signaling, others can inhibit it.

In fact, the Wnt signaling pathway is one of the most important in development and adult life of all multicellular organisms. The "canonical" Wnt signaling relies on reorganization of the Axin/APC/GSK3b destruction complex, whose function in the resting cell is to promote degradation of the cytoplasmic pool of b-catenin¹. In the presence of Wnt ligands, Frizzled (FZD) receptors and the co-receptor LRP5/6, through the joint action of the scaffolding protein Dishevelled and heterotrimeric G proteins^{2,3}, induce reorganization of the complex which results in stabilization of the cytoplasmic b-catenin, which can enter the nucleus and activate transcription of the Wnt target genes through interactions with TCF and other co-factors¹. Aberrant activation of the Wnt signaling pathway leads to tumorigenesis in many tissues⁴, including the breast⁵. Even historically, the first gene for a mammalian Wnt ligand (Wnt1) was identified as the integration point of the mouse mammary tumor virus (MMTV); MMTV insertion results in overexpression of Wnt1 and rapid development of lobuloalveolar hyperplasia followed by focal carcinomas⁶. Although the triple-negative breast cancer (TNBC) constitutes about 15% of all breast cancers, its death rate is disproportionally high. TNBC is highly aggressive and exhibits poor prognosis, being insensitive to the conventional targeted anti-breast cancer treatments due to absence of expression of the receptor tyrosine kinase HER2 and the estrogen receptor (ER)^{7,8}. Thus, development of novel therapies is imperative to combat TNBC. As in many other breast cancer cases, the β-catenin-dependent Wnt signaling is overly active in TNBC, being one of the causes of carcinogenesis. Unlike e.g. colon cancer, where mutations in downstream components of the Wnt/FZD pathway result in stabilization of b-catenin, breast cancers including TNBC typically stabilize b-catenin through activation of the pathway at the level of the ligand or receptor⁵. However, as several Wnt ligand proteins and their receptors of the FZD and LRP5/6 families are expressed in various cell types, identification of specific components of this pathway upregulated in TNBC is required before targeted interventions could be designed. Two recent studies identified FZD7⁹ and LRP6¹⁰ as the two co-receptors strongly overexpressed in TNBC over other breast cancer cells, characterizing them as potential drug targets.

In addition to the canonical signaling, Wnt can activate b-catenin-independent noncanonical pathways, such as the planar cell polarity signaling regulating the cytoskeleton¹¹, and the Wnt/Ca²⁺ pathway differently modulating other types of Wnt/FZD signaling¹². Although the separation of Wnt signaling into canonical and non-canonical branches is an oversimplification of complex context- and time-dependent cellular responses^{13,14}, there still exists a certain level of ligand specificity, which led to denomination of some Wnts (e.g. Wnt1, Wnt3a) as canonical and others as non-canonical (e.g. Wnt5a, Wnt7a). Non-canonical Wnts have in multiple instances been shown to oppose the action of the canonical ones¹⁴⁻¹⁶. Due to this property, the non-canonical FZD signaling is often viewed as tumor suppressive^{17,18}, although at later stages of cancer progression it may promote invasiveness and metastasis^{18,19}.

Autocrine Wnt production by TNBC cells is believed to be the reason for activation of FZD-LRP receptor complex, and blockade of Wnt-receptor interactions has been shown effective in preventing TNBC growth and metastasis in cell lines and xenograph models²⁰⁻²². A number of Wnt ligands are produced by normal and cancerous breast cells. By conventional RT-PCR, TNBC cells MDA-MB-468 and BT-20 have been reported to express multiple Wnts²³. Of those, Wnt3, Wnt3a, Wnt5a, and Wnt7a have been explicitly shown to activate the FZD7 protein²⁴⁻²⁶. The Wnt3-FZD7 interaction has been shown to trigger the canonical signaling²⁴, while Wnt7a binds FZD7 to activate the non-canonical pathway²⁶. In TNBC, FZD7 acts through the canonical β-catenin-dependent pathway²⁷. However, the identity of the Wnt protein activating FZD7 in TNBC remained unclear, imposing problems for development of specific anti-TNBC agents.

Currently there is no targeted therapy against TNBC, making this relatively rare form of the breast cancer (10-15% of cases) the dealiest type of cancer (>50% deaths). Application of Wnt7a-derived peptides will be such a therapy. Additional advantage of peptide-based drugs is their general low toxicity due to inherently high specificity.

However, therapeutic applications of the full length/whole Wnt7a protein may be troublesome due to various technical limitations.

Accordingly, it is one object underlying the present invention to provide for useful and effective means to treating cancer and in particular triple negative cancers. Another object is to provide for alternative treatment means that are easy to produce, to handle, to apply and stable in view of shelf life.

Various documents of the prior art relate to Wnt7a which are discussed in the following.

US 6,515,108 describes Wnt7a and its location at human chromosome 3p25. Also disclosed is a Wnt7a polypeptide and nucleic acid sequences and their use in treating various diseases as cancer, schizophrenia etc. as well as in diagnostics. The polypeptides of the current invention are neither disclosed nor suggested.

US2011/39788A2 describes a composition consisting of a LBH or LBH and native human Wnt7a protein mixture for the use in triple negative cancer. The polypeptides of the current invention are neither disclosed nor suggested.

WO 2013/040309 A2 describes a N-terminal deletion Wnt7a polypeptide and fusion proteins comprising same as well as vectors comprising a nucleic acid coding same and host cells. Moreover, their use in muscle degenerative diseases is claimed therein. The polypeptides of the current invention are neither disclosed nor suggested nor their use in cancer.

WO 2013/040341 A2 describes Wnt7a polypeptides and their use as stem cell modulator or in muscle diseases. The polypeptides of the current invention are neither disclosed nor suggested.

Wnt polypeptides have particular compound characteristics which render their production and purification difficult and imply a number of issues when handling these substances. Their amino acid sequence and composition make the polypeptides e.g. sticky. As a consequence it is difficult to handle these polypeptides and their application as medicament comes along with disadvantages, e.g. it is difficult to store and apply Wnt polypeptides as medicament.

One object underlying the present invention was also to provide for novel substances for the prevention or treatment of cancer, preferably Wnt-related cancers, in particular for triple cancers, or at least to ameliorate known cancer treatments or to avoid disadvantages of known cancer treatments.

### Summary of the Invention

In one aspect the invention relates to novel Wnt7a peptides as well as mixtures of two or more of such peptides.

In another aspect the invention relates to a composition comprising such Wnt7a peptides.

In another aspect the invention relates to such Wnt7a peptides and compositions thereof for use as a medicament.

In another aspect the invention relates to such Wnt7a peptides and compositions thereof for use in the prevention or treatment of Wnt-related cancers.

In another aspect the invention relates to methods for making the described Wnt7a peptides, mixtures thereof and compositions comprising same.

### Brief Description of the Figures

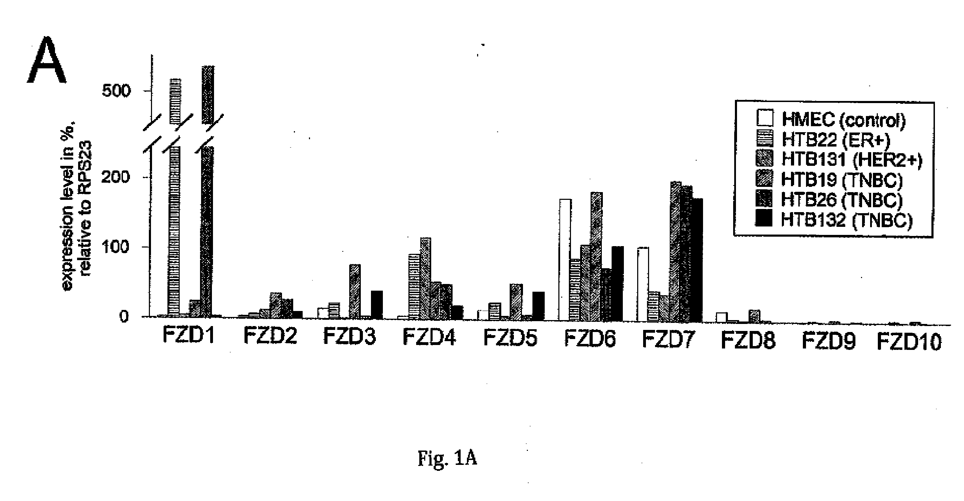

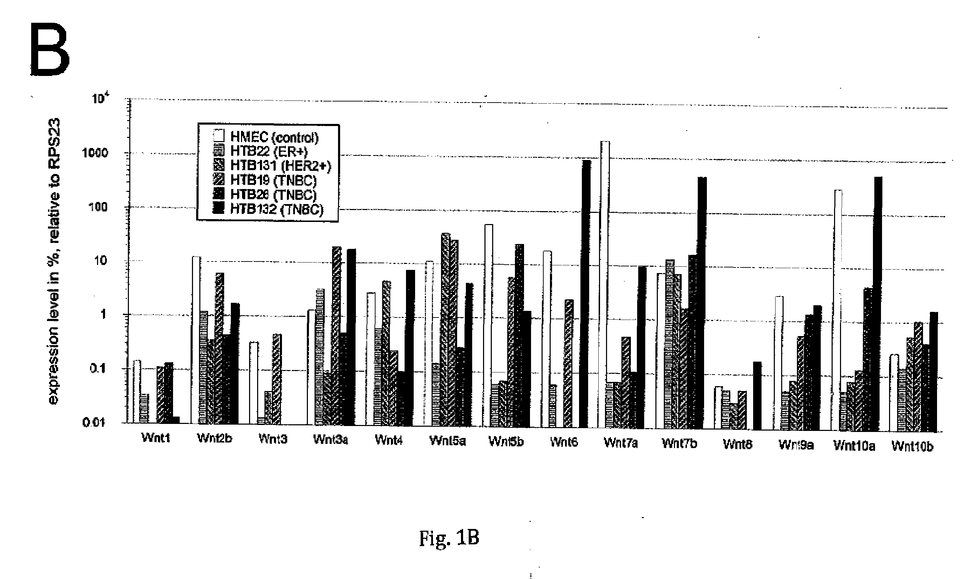

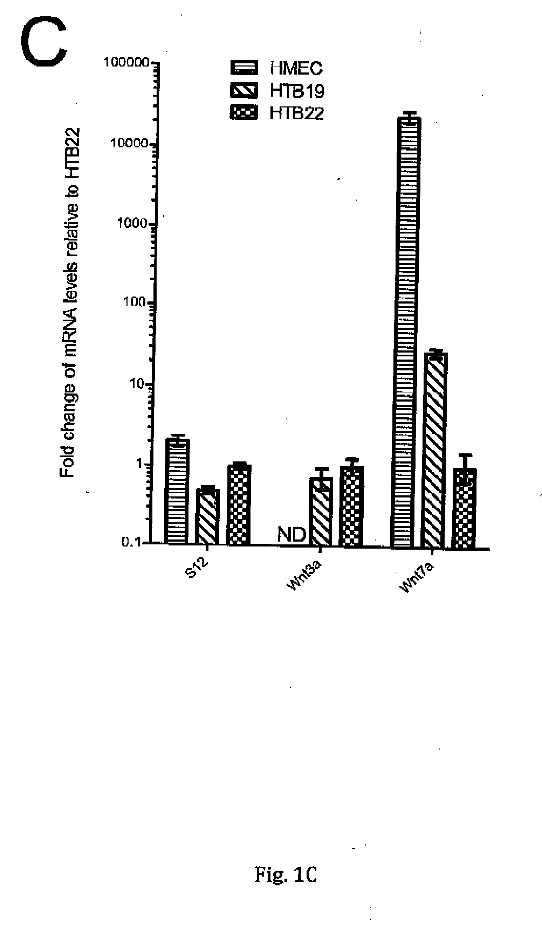

**Figure 1****. FZD and Wnt expression in healthy and cancer breast cell lines**. (A) Real-time RT-PCR analysis of FZD mRNA expression in normal and cancerous breast epithelium cell lines. Data are presented as levels of expression in percent of that of the ribosomal S23 and are mean of a triplicate experiment. FZD7 is overexpressed in all three TNBC lines. FZD1 is highly overexpressed in one TNBC and in one non-TNBC line. (B) Similar analysis of Wnt expression (note the logarithmic scale) shows that Wnt7a is overexpressed in normal breast epitelium (HMEC) and its expression drastically decreases in all the cancerous breast cell lines. Wnt3a is overexpressed in two of three TNBC cell lines. (C) Analysis of expression of selected Wnts (Wnt3a and Wnt7a) in selected breast lines (HTB-19 and HTB-22) as well as another healthy breast epithelia (telomerase-immortalized HMEC) using another house-keeping gene as a control (ribosomal protein RPS12) confirms the data of experiment (B). Note that the data are presented as % of expression in HTB-22 cells.

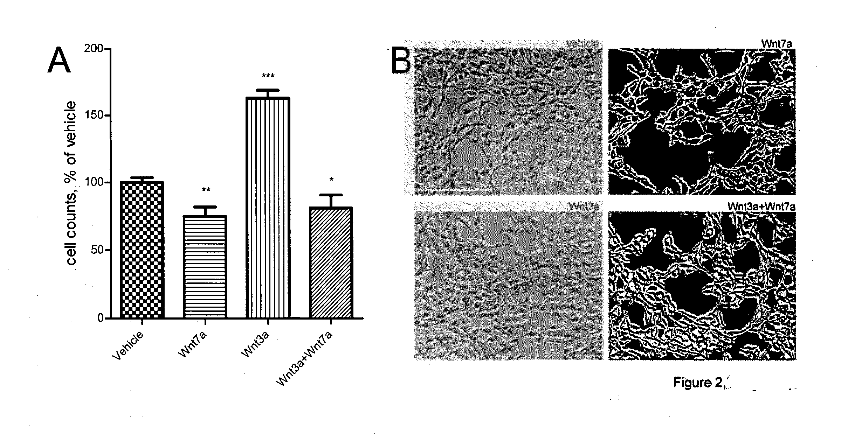

**Figure 2****. Inhibition of TNBC cell growth by Wnt7a treatment. (A)** HTB-19 cells were grown for 4d in the presence of indicated treatments (freshly added each day). Wnt3a enhances growth, while Wnt7a impedes proliferation. The error bars show standard deviation. (B) Examples of morphological examinations of HTB-19 cells grown in the presence of different treatments. Note that cells in the presence of Wnt7a appear more clustered.

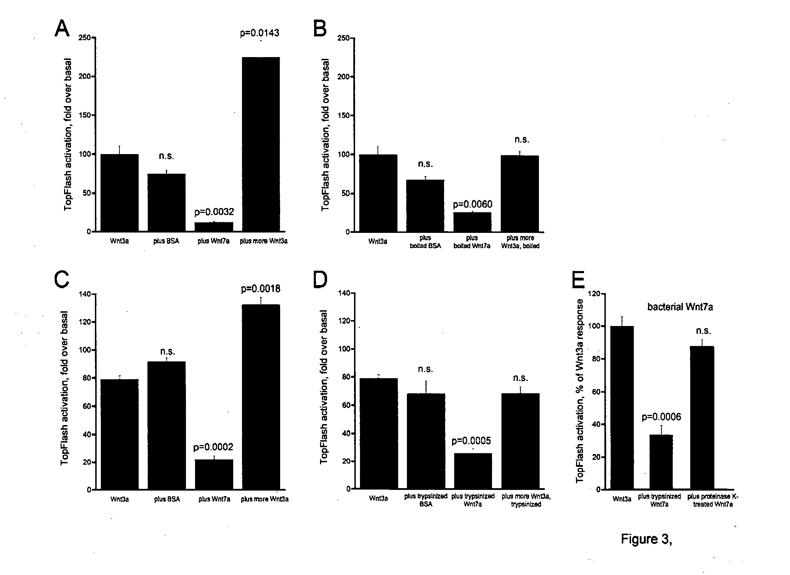

**Figure 3****. TopFlash assay in TNBC HTB-19 cells.** (A) 250ng/ml Wnt3a induces massive activation of TCF-dependent transcription, inhibited by Wnt7a (700 ng/ml) but not BSA. Addition of equivalent amounts of extra Wnt3a further activates the assay. (B) Heat-denaturation of the three extra-added proteins shows that Wnt3a activity is heat-labile, while that of Wnt7a is heat-resistant. (C, D) Equivalent experiment investigating trypsin-sensitivity of the three extra proteins similarly shows that Wnt3a is sensitive, and Wnt7a - resistant to trypsinization. (E) bacterially produced Wnt7a upon trypsinization also can reliably inhibit Wnt3a-induced TopFlash activation in HTB-19 cells, but proteinase K-treated Wnt7a cannot.

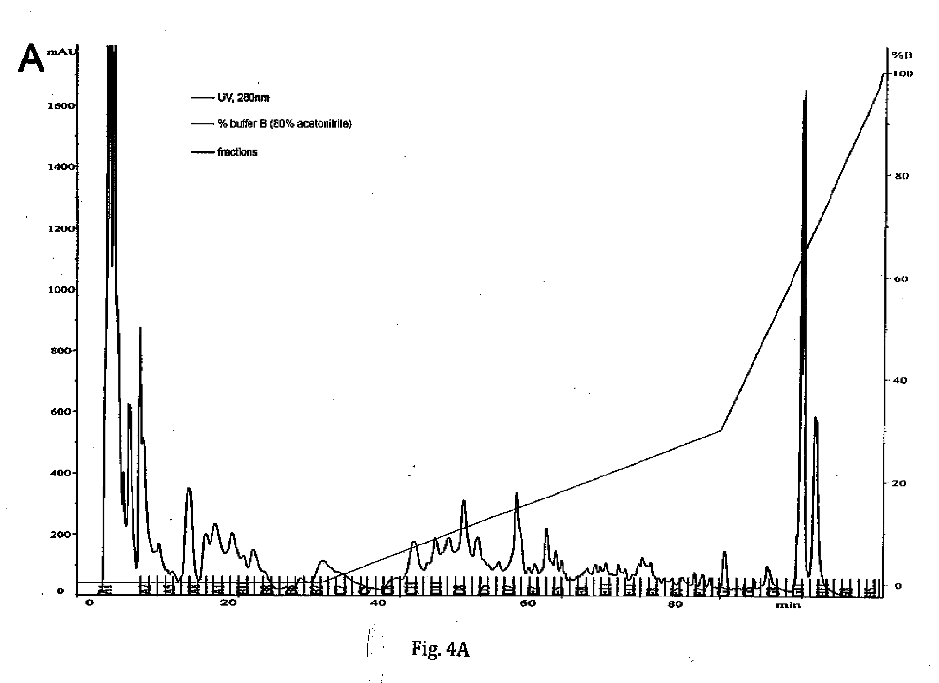

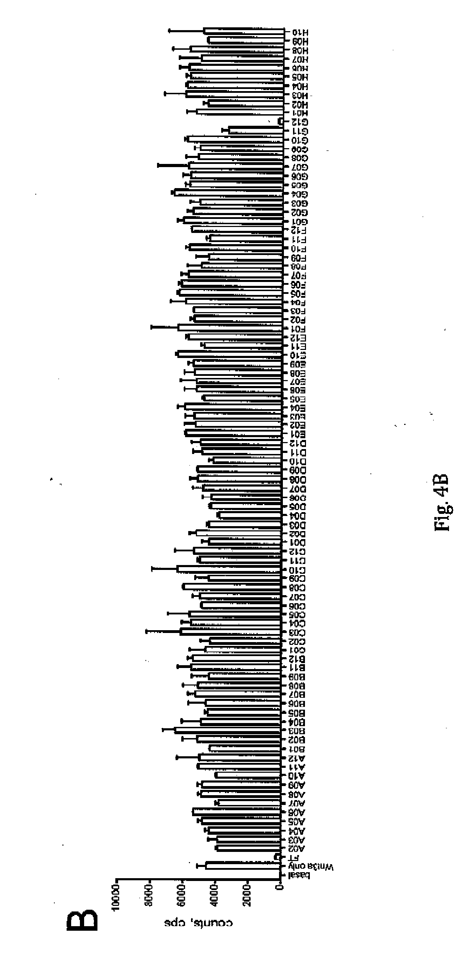

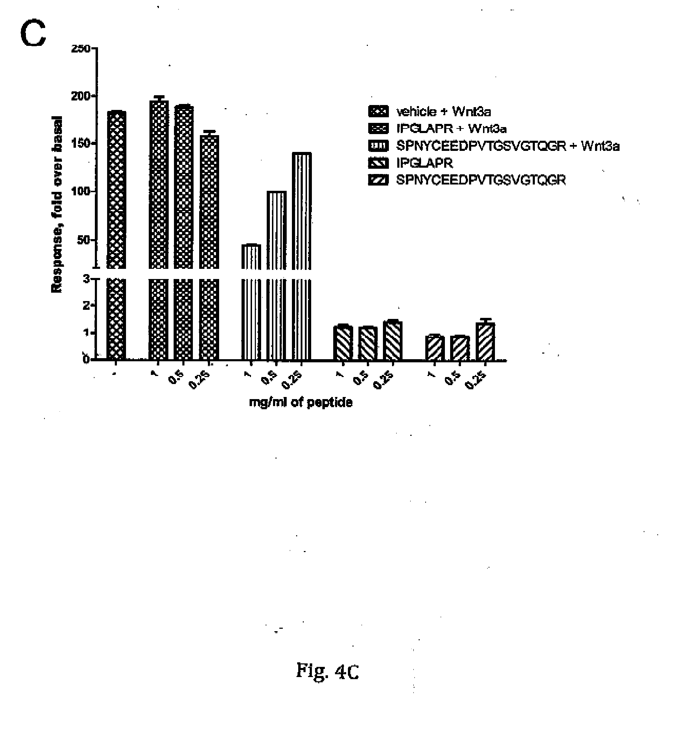

**Figure 4****. Identification of the Wnt7a trypsinization-derived peptide mediating the Wnt3a-inhibiting activity.** (A) HPLC separation of the bacterially produced and trypsinized Wnt7a. (B) Analysis of fractions for the inhibitory activity identifies fraction G12 as the one possessing the activity. (C) Analysis of the two synthetic peptides corresponding to those identified in fraction G12 by mass-spectrometry shows that peptide SPNYCEEDPVTGSVGTQGR but not IPGLAPR possesses a concentration-dependent activity suppressing Wnt3a-induced activation of canonical Wnt signaling in TNBC HTB-19 cells.

### Detailed Description of the Invention

The invention provides for Wnt7a peptides and their medical use in general and in particular their use in cancer treatment.

In particular the invention relates to one or more polypeptides wherein the polypeptide is a fragment of a Wnt7a polypeptide, preferably it is a human sequence. In a preferred embodiment the invention relates to such polypeptides according to Table 1.

In the following certain terms of the invention will be defined and unless otherwise stated the terms of the invention are to be understood accordingly.

"Nucleic acid" or "polynucleotide" as used herein refers to purine- and pyrimidine-containing polymers of any length, either polyribonucleotides or polydeoxyribonucleotide or mixed polyribo-polydeoxyribonucleotides. This includes single-and double-stranded molecules, i.e., eDNA, mRNA, DNA-DNA, DNA-RNA and RNA-RNA hybrids, as well as "protein nucleic acids" (PNA) formed by conjugating bases to an amino acid backbone. This also includes nucleic acids containing modified bases.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester anologs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules (e.g., restriction fragments), plasmids, and chromosomes. In discussing the structure of particular double-stranded DNA molecules, sequences may be described herein according to the normal convention of giving only the sequence in the 5'to 3' direction along the nontranscribed strand of DNA (i.e., the strand having a sequence homologous to the mRNA). A "recombinant DNA molecule" is a DNA molecule that has undergone a molecular biological manipulation.

The term "polypeptide" or "peptide", in accordance with the present invention, describes linear molecular chains of amino acids, including single chain polypeptides or their fragments, containing more than 5, preferably more than 6, 7, 8, more preferably more than 10 amino acids. The term "fragment" in accordance with the present invention refers to a portion of the polypeptide comprising at least the amino acid residues necessary to maintain the biological activity of the polypeptide. Polypeptides may further form oligomers consisting of at least two identical or different molecules. The corresponding higher order structures of such multimers are, correspondingly, termed homo- or heterodimers, homo- or heterotrimers etc. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" are used interchangeably herein and also refer to naturally modified polypeptides wherein the modification is effected e.g. by glycosylation, acetylation, phosphorylation and the like. Such modifications are well known in the art.

An "amino acid" in the sense of the invention can be any known naturally or synthetically produced amino acid. The amino acid can be further modified by known techniques including e.g. methylation.

The terms "identical" or percent "identity," or "amino acid identity" in the context oftwo or more nucleic acids or polypeptide sequences, refer to two or more sequences or subsequences that are the same or have a specified percentage of amino acid residues or nucleotides that are the same (i.e., about 70% identity, preferably 75%, 80%, 85%, 86%, 87%, 88%, 89%, 90%, 91%, 92%, 93%, 94%, 95%, 96%, 97%, 98%, 99%, or higher identity over a specified region (e.g., epitope), when compared and aligned for maximum correspondence over a comparison window or designated region) as measured using e.g. a BLAST sequence comparison algorithms, or by manual alignment and visual inspection. Such sequences are then said to be "substantially identical." The definition also includes sequences that have deletions and/or additions, as well as those that have substitutions. As described below, the preferred algorithms can account for gaps and the like. Preferably, the identity exists over a region that is at least about 25 amino acids or nucleotides in length, or more preferably over a region that is 50-100 amino acids or nucleotides in length, or more preferably over the complete length of said two or more nucleic acids or polypeptide sequences.

In the sense of the invention a "derivative" or "mutant" is characterized by changes in nucleic acid sequence or amino acid sequence wherein single nucleotides or amino acids are changed or modified by insertion, deletion, replacement, or modification well known in the art.

As used herein, the term "fragment" or "polypeptide" or "peptide" of Wnt7a according to the invenion refers to a peptide or polypeptide comprising an amino acid sequence of at least 2 contiguous amino acid residues, at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, at least 200 contiguous amino acid residues, at least 250 contiguous amino acid residues, at least 300 contiguous amino acid residues, at least 300 contiguous amino acid residues, at least 500 contiguous amino acid residues, at least 750 contiguous amino acid residues, at least 1000 contiguous amino acid residues, or between 6 to 75 contiguous amino acid residues, between 25 to 150 contiguous amino acid residues, or between 25 to 300 contiguous amino acid residues of the amino acid sequence of a peptide, polypeptide or protein As used herein, the term "fragment" in the context of a nucleic acid refers to a nucleic acid comprising an nucleic acid sequence of at least 2 contiguous nucleotides, at least 5 contiguous nucleotides, at least 10 contiguous nucleotides, at least 15 contiguous nucleotides, at least 20 contiguous nucleotides, at least 25 contiguous nucleotides, at least 30 contiguous nucleotides, at least 35 contiguous nucleotides, at least 40 contiguous nucleotides, at least 50 contiguous nucleotides, at least 60 contiguous nucleotides, at least 70 contiguous nucleotides, at least contiguous 80 nucleotides, at least 90 contiguous nucleotides, at least 100 contiguous nucleotides, at least 125 contiguous nucleotides, at least 150 contiguous nucleotides, at least 175 contiguous nucleotides, at least 200 contiguous nucleotides, at least 250 contiguous nucleotides, or between 8 to 75 contiguous nucleotides, between 25 to 150 contiguous nucleotides, or between 25 to 300 contiguous nucleotides of the nucleic acid sequence encoding a peptide, polypeptide or protein.

A "vector" is any means for the transfer of a nucleic acid into a host cell. A vector may be a replicon to which another DNA segment may be attached so as to bring about the replication of the attached segment. A "replicon" is any genetic element (e.g., plasmid, phage, cosmid, chromosome, virus) that functions as an autonomous unit of DNA replication in vivo, i.e., capable of replication under its own control. The term vector includes both viral and non-viral means for introducing the nucleic acid into a cell in vitro, ex vivo or in vivo. Viral vectors include alphavirus, retrovirus, adena-associated virus, pox, baculovirus, vaccinia, herpes simplex, Epstein-Barr and adenovirus vectors. Non-viral vectors include, but are not limited to plasmids, liposomes, electrically charged lipids (cytofectins), DNA-protein complexes, and biopolymers. In addition to a nucleic acid, a vector may also contain one or more regulatory regions, and/or selectable markers useful in selecting, measuring, and monitoring nucleic acid transfer results (transfer to which tissues, duration of expression, etc.).

A "host cell" in the sense of the invention can be any prokaryotic or eukaryotic cell. Such cells can be applied for polypeptide expression and production of the polypeptides of the invention.

Non-human hosts according to the present invention can be single cells or multi-cellular organisms. Suitable prokaryotic hosts comprise e.g. bacteria of the species Escherichia, Streptomyces, Salmonella or Bacillus. Suitable eukaryotic host cells are e.g. yeasts such as Saccharomyces cerevisiae or Pichia pastoris or chicken cells, such as e.g. DT40 cells. Insect cells suitable for expression are e.g. Drosophila S2 or Spodoptera Sf9 cells. Suitable zebrafish cell lines include, without being limiting, ZFL, SJD or ZF4.

Mammalian host cells that could be used include, human Hela, HEK293, H9 and Jurkat cells, mouse NIH3T3 and C127 cells, COS 1, COS 7 and CV1, quail QC1-3 cells, mouse L cells, mouse sarcoma cells, Bowes melanoma cells and Chinese hamster ovary (CHO) cells. Also within the scope of the present invention are primary mammalian cells or cell lines. Primary cells are cells which are directly obtained from an organism. Suitable primary cells are, e.g., mouse embryonic fibroblasts (MEF), mouse primary hepatocytes, endothelial cells, cardiomyocytes and neuronal cells as well as mouse muscle stem cells (satellite cells) and stable, immortalized cell lines derived thereof. Alternatively, the recombinant polypeptide of the invention can be expressed in stable cell lines that contain the gene construct integrated into a chromosome.

Appropriate culture media and conditions for the above described host cells are known in the art.

For molecular biology techniques, see Sambrook and Russel "Molecular Cloning, A Laboratory Manual", Cold Spring Harbor Laboratory, N.Y. (2001) which is incorporated by reference herewith.

The terms "treating", "treat" and "treatment" include (i) preventing a disease, pathologic or medical condition from occurring (e.g., prophylaxis);(ii) inhibiting the disease, pathologic or medical condition or arresting its development;(iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, veterinary, therapeutic, and/or prophylactic administration, as appropriate.

The term "modulator", "modulating" or "to modulate" in the sense of the invention is to be understood as inhibiting or activating a cellular process or cascade which will lead to an effect in the activity, expression or otherwise in a cell or an organism and which may result in change of the phenotype of a cell or organism or lead to a physiological change or otherwise and which is measurable and/or can be made visible with means generally known in the art.

The terms "inhibit", "inhibiting", and "inhibition" refer to the slowing, halting, or reversing the growth or progression of a disease, infection, condition, or group of cells. The inhibition can be greater than about 20%, 40%, 60%, 80%, 90%, 95%, or 99%, for example, compared to the growth or progression that occurs in the absence of the treatment or contacting. Accordingly and in an equivalent manner the term "activate", "activating", and "activation" is to be understood.

The nucleic acid and polypeptide molecules of the invention can be made with known methods of synthesis or recombinant technology well known in the art. Such methods include without limitation chemical synthesis and expression of polypeptides by application of recombinant technologies.

A "composition" or "pharmaceutical composition" according to the invention contains or comprises a polypeptide according to the invention and will be formulated according to the state of the art. Such a composition may contain additional carriers, auxiliaries and/or adjuvants well known in the art.

The invention in one aspect relates to peptides that can be provided as single peptides or as a mixture of peptides comprising or consisting of components listed in Table 1.

The peptides can be produced by known methods, e.g. they can be made from human Wnt7a digestion by trypsin, or by peptide synthesis methods known in the art or recombinant techniques.

Advantageously the peptides, preferably a mixture of peptides, is suppressing Wnt3a-induced response in the TNBC (triple-negative breast cancer) cells.

In alternative embodiments of the invention the peptides of the invention or a mixture thereof may inhibit Wnt1-induced responses or even inhibit responses induced by other Wnt ligands.

In an embodiment of the invention the peptides of the invention as listed in Table 1 combined in one mixture, or a selection of any of the peptides listed as two, three, four, or five peptides selected from those listed in Table 1, thus can be used for treatment of this type of cancer as well as for other Wnt-dependent cancers such as lung, liver and colorectal cancers.

It was surprising that the peptides or a mixture of the peptides of Table 1 is active when obtained both by digestion of polypeptide produced in human cells as well as other sources, such as Escherichia coli. The mixture can be also produced from separate components.

Peptides according to the invention resulting from the digestion of native sources, e.g. human full length Wnt7a polypeptide may contain naturally occurring modifications. Such modifications can also be introduced with known techniques in synthetically produced peptides of the invention.

Such production methods include the respective host cells and vectors as well as the nucleic acid molecules coding for the respective polypeptides according to the invention.

Any amino acid of the peptides or mixture according to the invention might be modified through known biological process (e.g. N-, C- or O-linked glycosilation, methylation, acylation, amidation, hydroxilation, sulfation etc, see http://dbptm.mbc.nctu.edu.tw/index.php for full list of possible protein modifications).

In a alternative embodiment and depending on the modifications as the result of such native or chemically induced modification, peptides listed in Table 1 may also form covalently or non-covalently bound oligomers (e.g. persulfide bond, ionic interactions etc.) which may contain any number of mixture components.

Though the components in the mixture produced by digestion will be present at a close to equimolar amount of each, any other ratios (including 0 - i.e. omitting any amount of any of them) may possess same or improved inhibitory activity.

Any of above mentioned naturally occurring modifications of the mixture or alteration of the mixture composition might be also introduced by artificial means (e.g. chemical process) to modify the activity of the peptides of the invention and of a mixture according to the invention.

Additionally, common modifications applicable for peptides to improve their medicinal chemistry might be applied to one or more components of the mixture. Such modifications may serve several purposes:
1) increasing existing peptide stability, such as changes of amino acids at dispensable positions (up to 75% remaining identity to original sequence), replacement of naturally occurring amino acids with their analogs, cyclization (both complete, using the terminal amino acids, and partial, involving amino acids inside the sequence), formation of pseudopeptide bonds (e.g. ether, alkene, thioester etc) between amino acids and incorporation of D-amino acids.
2) increasing stability of the active structure by synthesis of peptidomimetics of non-amino acid nature, but which structure could be rationally inferred from the sequence of any component of the mixture in Table 1 using them as templates.

Such mimetics will consist of elements which contain functional group identical or similar to the one existing at corresponding position of template peptide. Examples of such are sulfonamide-, sulfamate- and pyrrolinone-based peptidomimetics
3) improvements of the PKPD (pharmacokinetics-pharmacodynamics) characteristics of the peptides or peptide-based mimetics, such as bioavailability or distribution, through attachment of functional or protective groups to the termini or side chains of amino acids. The same could be achieved through fusions with carrier proteins or delivery agents.

Within the scope of the invention there are also other peptide-derivatives of Wnt7a which may partially overlap with those listed in Table 1.

Embodiments of the invention also encompass modified peptides or variants achieved by e.g. peptide walking. In particular synthesizing peptides 'shifted' in their sequence upstream or downstream from the ones listed in Table 1. Such peptides according to the invention comprise embodiments which are derivatives of Wnt7a but may coincide with those sequences listed in Table 1 only e.g. by 70% to 90%, or 75% to 85%, or 75% - the remaining percentage being Wnt7a sequences up- or downstream from the sequence in the Table 1.

The peptides listed in Table 1 can be produced from Wnt7a after trypsinization or comparable methods as described herein or known to the skilled person. It was surprising that the peptides according to the invention or a mixture thereof indeed recapitulates the effects of whole Wnt7a. More so, the inventors in this manner achieved additional advantages which make the inventive peptides, mixtures thereof, compositions comprising same and their use so superior to the prior art. Finally this provides for an easy and efficient handling of these peptides, their preparation and their medical applications and uses.

In alternative and/or preferred embodiments the invention relates to a polypeptide according to the invention wherein the polypeptide is characterized by one or more modifications of one or more amino acids of the polypeptide.

The amino acid modification of the polypeptide of the invention can be any known in peptide and protein chemistry and is preferably a N-, C- or O-linked glycosylation, methylation, acylation, amidation, hydroxilation, or sulfation.

One or more polypeptides, preferably one, tow, three, four, five, six, seven, eight, nine, ten, eleven, twelve polypeptides are combined, preferably from the polypeptides listed in Table 1. Particularly useful are any peptides 1 to 29 of Table 1 or any combinations thereof wherein the following combinations can show advantageous efficacy: 1 to 10, 11 to 20, 21 to 29, 2 to 8, 12 to 19, 22 to 28, 5 to 15, 16 to 26, 27 to 29, 1 to 3, 4 to 6, 7 to 9, 10 to 13, 14 to 17, 18 to 21, 22 to 24, 25 to 27, or 28 to 29. The usefulness of he peptide combinations can be assessed by known experimentation.

In another aspect the invention relates to a composition comprising one or more polypeptides according to the invention.

Such a composition according to the invention may contain or comprise at least two polypeptides covalently linked to each other.

The polypeptide or composition according to the invention can be applied for medical use.

In a particular application the polypeptide(s) or composition according to the invention is useful for use in preventing or treating cancer. Preferred cancer types are any Wnt-dependent cancer or/and a triple-negative breast cancer.

The use of the peptides or the composition of the invention implies a number of advantages. One advantage is that in contrast to the full length Wnt7a polypeptide which is sticky and difficult to handle in a medical setup or a pharmaceutical formulation the polypeptides of the invention can easily be formulated and handled in production and medical applications.

The inventors could show that healthy breast epithelia massively produce one of such inhibitory Wnt proteins, Wnt7a, and that upon cancerous transformation expression of Wnt7a is dramatically lost, cordinated by overproduction of canonical Wnts and their receptors. The inventors could further show that Wnt7a is able to suppress the canonical Wnt signaling as well as proliferation in TNBC cells, stimulated by the pro-oncogenic Wnt3a protein. These findings identify Wnt7a as an anti-breast cancer drug or a lead candidate for further molecule optimization. The inventors could find that the Wnt-inhibitory and anti-TNBC activity of Wnt7a could be recapitulated by the peptides derived from it upon the proteolytic digestion by Trypsin. Remarkably, not only eucaryotically expressed Wnt7a, but also the protein derived from bacteria upon recombinant expression can be a reliable source of tumour-suppressive peptides after trypsinization, or equally synthetically produced peptides according to the invenion.

### Examples

In the following the invention will be illustrated by way of examples which shall not be construed as limiting in any way but are meant to serve as exemplary embodiments of the invention only.

In this study we analyzed the expression profiles of Wnt ligands and their receptors Frizzled (FZD) in normal mammary epithelial cells, as well as in TNBC and non-TNBC breast cancer cell lines. We confirm the existing data by demonstrating the overexpression of FZD7 in all analyzed TNBC cell lines, which, in combination with Wnt3a or other canonical Wnt ligand overexpression, is the probable reason for transformation in most triple-negative breast tumors. We also observe an extremely high overexpression of the non-canonical ligand Wnt7a in normal breast epithelium cells, which drastically decreases in all breast cancer lines tested. We propose a "molecular shield" role for Wnt7a, which competes with canonical Wnt ligands, "guarding" the FZD proteins from abnormal stimulation, and thus protecting the breast tissue from malignization. We also formulate a noncanonical-to-canonical switch in Wnt signaling as a possible general mechanism of breast transformation. We show that Wnt7a treatment inhibits TNBC and non-TNBC (but BC) cell proliferation in culture and blocks the canonical Wnt3a-induced signaling in TNBC cells, demonstrating the actual anti-cancer role of the Wnt7a ligand and its natural competition with a canonical Wnt-ligand. Moreover, we demonstrate the ability of denatured, bacterially produced, and even trypsin-treated Wnt7a to inhibit the TNBC cell growth and the canonical Wnt cascade therein, suggesting that epitope(s) different from those required in canonical signaling mediate the effects of Wnt7a in breast epithelia. These findings led us to identify Wnt7a-derived peptides as lead compounds for the anti-breast cancer drug discovery.

### Experimental results

To complete the Wnt-FZD expression pattern in cancerous and in normal breast cells, we analyzed the expression pattern of 14 Wnt ligands previously reported to be present in breast epithelial (normal or cancerous) cells²³ and 10 FZD receptors in different breast cancer cell lines and in the control mammary epithelium line HMEC. The cancer lines chosen represented TNBC (lines HTB-19, HTB-26 and HTB-132), ER-positive breast cancer (line HTB-22) and HER2-positive breast cancer (line HTB-131) The expression levels were determined by real-time PCR with reverse transcription, with ribosomal protein S23 as a standard, which is much more sensitive than the previous conventional RT-PCR study²³. Although each tested line shows distinct Wnt and FZD expression pattern (Figure 1), some generalizations can be drawn from this analysis.

First, we find overexpression of FZD7 in all three TNBC lines, confirming the previous findings about the role for this receptor in TNBC⁹. However, we also find a potential importance of other receptors, as FZD1 is also found to be heavily overexpressed in two cancer cell lines (HTB-22 and HTB-26), the first being an ER-positive line, and the second - a TNBC line (Figure 1A).

Second, a significant overexpression of the canonical Wnt3a ligand is observed in two of three TNBC lines, identifying this ligand as the likely reason for the autocrine activation of canonical Wnt signaling in a subset of TNBC lines. Wnt5b, acting as a canonical ligand in some contexts²⁸, may be implicated in the proliferation of the remaining TNBC line HTB26 (Figure 1B). If so, Wnt5b might act through FZD1, highly expressed in these cells (Figure 1A). FZD1, but not FZD7, has previously been shown to interact with Wnt5b²⁵.

And third - we find an unexpectedly high expression of the non-canonical ligand Wnt7a in the normal mammary epithelium line HMEC (Figure 1A) as well as another HMEC cell line immortalized by telomerase transfection (Figure 1C). In contrast, the Wnt7a expression was drastically downregulated in all of the examined cancer cell lines, regardless if they were TNBC or not (Figure 1B, C). This is not the case for another non-canonical Wnt, Wnt5a (Figure 1), which has been described previously to be down-regulated in some breast cancer lines²⁹, and whose re-expression could serve to suppress breast cancer cell migration³⁰.

Wnt3a and Wnt7a are both known to bind and activate FZD7, although Wnt3a has an apparently higher affinity for the receptor²⁵. Importantly, the two Wnts activate different types of signaling on FZD7: Wnt7a activates the non-canonical²⁶ and Wnt3a - the canonical signaling²⁷. Given the many examples of the antagonistic action of non-canonical Wnt ligands on activation of the canonical Wnt signaling^{14-16,28}, we propose the following model as one of the driving forces of TNBC transformation: i) Wnt7a, abundant in healthy breast epithelia, activates non-canonical FZD7 signaling and suppresses the canonical pathway; ii) loss of Wnt7a expression is required for breast cancer transformation/progression; iii) Wnt3a is up-regulated in some TNBC lines to activate canonical signaling through FZD7; iv) in a minority of TNBC lines, Wnt5b, acting through FZD1, may provide the alternative route of activation of canonical Wnt signaling. Thus, we propose a noncanonical-to-canonical switch in Wnt signaling in the breast as a likely general mechanism behind cancerous transformation. The reversal of this switch by re-introduction of Wnt7a may become an attractive approach to develop anti-TNBC treatments.

To directly test our hypothesis about the putative anti-carcinogenic role of Wnt7a in the breast tissue, we examined the effects of Wnt7a on TNBC cell proliferation. HTB-19 cells were treated with Wnt7a or the control CHAPS-containing buffer during four days. We found a significant decrease in the growth rate of all three breast cancer cell lines upon Wnt7a treatment (Figure 2A).

Ectopic stimulation of the canonical Wnt signaling has been shown to further boost breast cancer cell proliferation⁹. In agreement with that, we see that ectopic Wnt3a promotes proliferation of HTB-19 cells (Figure 2A). Importantly, addition of Wnt7a could also abrogate this Wnt3a-induced growth stimulation (Figure 2A). Morphological examination of HTB-19 cells grown in different conditions reveals another interesting effect of Wnt7a treatment: in the presence of this ligand, remaining TNBC cells appear more clustered, which may reflect their return to a more epithelial and less mesenchymal phenotype.

Thus, these data cumulatively led us to conclude that Wnt7a indeed acts as an inhibitor of TNBC cell proliferation.

The data presented above show that Wnt7a is capable of suppression of breast cancer cell proliferation. To elucidate if this effect is paralleled by a suppression of canonical - β-catenin dependent - Wnt signaling, as has been demonstrated in other TNBC cell studies^{9,31}, we performed the TopFlash assay in HTB-19 cells. We found that addition of Wnt3a strongly stimulated β-catenin signaling: ca. 100-fold stimulation of TCF-dependent transcription is seen in HTB-19 cells upon addition of 250ng/ml Wnt3a, and further increased if this concentration is increased (Figure 3A, C). As expected, this effect of Wnt3a is completely reversed by co-treatment with Wnt7a, but not BSA (Figure 3A, C).

Wnts are secreted proteins of ca. 40kDa, bearing multiple post-translational modifications, namely glycosilations and lipidations, required for the biological activity, making it impossible, according to the current knowledge, to produce active Wnts in non-eukaryotic hosts³². A recent crystal structure of a Xenopus Wnt8 reveals an unusual fold with two distinct sites responsible for the proper interaction with the FZD receptor³³. We were therefore surprised to realize that heat-denaturation of Wnt7a did not eliminate its activity in suppressing TNBC cell growth (not shown) or Wnt3a-induced activation of the canonical signaling - while a similar treatment of Wnt3a completely abolished Wnt3a's activities (Figure 3A, B).

Remarkably, trypsin digestion similarly did not remove the ability of Wnt7a to suppress Wnt3a-induced signaling, while trypsinization of Wnt3a itself abrogated its activity (Figure 3C, D). Finally, we further showed that Wnt7a, recombinantly produced in bacteria and trypsin-digested, also retains its signaling-suppressive activity (Figure 3E). Importantly, treatment of Wnt7a with proteinase K (which shreds proteins in smaller pieces than trypsin does) eliminated the activity of the ligand (Figure 3E). These data suggest that the mechanism of Wnt7a-mediated growth suppression and suppression of the canonical signaling are distinct from those of Wnt3a-mediated activation, and are provided by modifications-independent peptide(s) within the Wnt7a sequence.

We next aimed at identifying the Wnt7a peptide(s) mediating the anti-TNBC activities of the intact protein. To this end we trypsinized bacterially produced Wnt7a and separated the peptide mixture through HPLC (Figure 4A). Fractions eluted with a gradient of acetonitrile were collected and tested for their ability to suppress Wnt3a-induced TopFlash activation in HTB-19 cells. The inhibitory activity was concentrated in a single fraction - fraction G12 (Figure 4B). Mass-spectrometry of the peptides present in the active fraction but absent in the neighboring ones identified two candidate peptides: IPGLAPR and SPNYCEEDPVTGSVGTQGR (corresponding to the amino acids 41-47 and 274-292 of the Wnt7a sequence, respectively).

These two peptides were synthesized and their activities against Wnt3a-induced TopFlash activation in HTB-19 cells tested. Figure 4C shows that the IPGLAPR peptide reveals no activity, while the SPNYCEEDPVTGSVGTQGR peptide inhibits the canonical signaling in a concentration-dependent manner. The non-canonical, PCP-type activity of Wnt7a in promoting satellite cell expansion (the process where Wnt7a also interacts with FZD7²⁶) has recently been shown to be recapitulated by a C-terminal half of the protein lacking lipid modifications³⁴. Our experiments identify a much smaller peptide within the C-terminal region of Wnt7a which can recapitulate the protein's activities against breast cancer; it is conceivable that the same peptide may also be active in muscle stem cell proliferation. We also would like to add that the peptide we identified (SPNYCEEDPVTGSVGTQGR) may be not the only sequence suppressing TNBC; peptides including this sequence but possessing flanking amino acids may prove to be even more active; this work is in progress now.

In summary, we discover a dramatic loss of expression of Wnt7a upon cancerous transformation in the breast, concomitant with overexpression of Wnts and FZDs which stimulate pro-proliferative canonical signaling in this tissue, providing the basis for the hypothesis of the noncanonical-to-canonical switch as the driving force of breast malignization. Re-introduction of Wnt7a reverses TNBC cell proliferation and canonical signaling, identifying this protein as a molecular shield against breast cancer. However, therapeutic applications of the whole Wnt7a would be limited. Instead, we further pinpointed peptide sequences within Wnt7a which recapitulate the anti-breast cancer activities of the whole protein. These peptides, their derivatives and stabilized analogs are highly promising drug candidates to treat breast cancer and especially TNBC - its deadliest and most incurable form.

### Materials and methods of preferred embodiments

### Cell cultures

All cancer cell lines (HTB22, HTB131, HTB19, HTB26, HTB132, all from ATCC) were routinely maintained in DMEM/10% FCS (PAA). Both primary HMEC from Lonza (CC-2551) and immortalized HMEC 184DTERT cell^{35,36} were maintained in MEGM (MEBM supplemented with 5ng/ml EGF, 0.5µg/ml hydrocortisone, 5µg/ml insulin, 70µg/ml BPE (SingleQuotes, Lonza CC-4136). For proliferation assay, HTB19 cells were incubated in DMEM/2% FCS for 4 days in presence of indicated amounts of agents; the medium was replaced with the fresh one supplemented with the same concentrations once every 24 hours. Cells were subsequently quantified using CCK-8 kit (Sigma) according to the manufacturer's protocol.

### Reverse transcription and qPCR

The total RNA was extracted from cells in one confluent well in the 6-well plate using Trizol reagent (Life Technologies) according to the manufacturer's protocol. The reverse transcription was performed with TAKARA RNA PCR kit (RR019B) with Xxµg of total RNA using oligodT primer according to the manufacturer's protocol. The obtained cDNA was used directly in qPCR using the TaqMan Gene Expression master mix, Applied Biosystems (4369016) and following TaqMan Gene Expression Assay primer/probe sets: FZD1 (Hs00268943_s1), FZD2 (Hs00361432_s1), FZD3 (Hs00184043_m1), FZD4 (Hs00201853_m1), FZD5 (Hs00258278_s1), FZD6 (Hs00171574_m1), FZD7 (Hs00275833_s1), FZD8 (Hs00259040_s1), FZD9 (Hs00268954_s1), FZD10 (Hs00273077_s1); Wnt1 (Hs01011247_m1), Wnt2b (Hs00921614_m1), Wnt3 (Hs00229135_m1), Wnt3a (Hs00263977_m1), Wnt4 (Hs01573504_m1), Wnt5a (Hs00998537_m1), Wnt5b (Hs01086864_m1), Wnt6 (Hs00362452_m1), Wnt7a (Hs01114990_m1), Wnt7b (Hs00536497_m1), Wnt8b (Hs00610126_m1), Wnt9a (Hs00243321_m1), Wnt10a (Hs00228741_m1), Wnt10b (Hs00559664_m1), RPS23 (Hs01374150_g1), RPS12 (Hs00831630_g1) (all from Invitrogen) in the ABI 7500 Fast qPCR system.

### Luciferase-based assay of the Wnt-dependent transcriptional activity

The assay was performed in white tissue-culture treated 96-well plates (Greiner BioOne, cat.# 655073) and either HTB19 cells transiently transfected with mixture of M50 Super 8x TOPFlash (Addgene, plasmid 12456) and pCMV-RL (kindly provided by Konrad Basler³⁷ plasmids or HEK293T cells stably transfected with M50 Super 8x TOPFlash plasmid only. In both cases cells were seeded in 100µl DMEM medium containing 10% FCS at ~5000 cells/well and subsequently stimulated by indicated concentrations of Wnt ligands (either commercially available Wnt7a, (R&D Systems, 3008-WN-025/CF) or purified as described³⁸, or other specified agents for 12-24 hours.

Transfection was performed using X-tremeGENE 9 reagent (Roche) according to the manufacturer's protocol. The medium was subsequently removed, and 15µl of the lysis buffer (25mM Glycylglycine pH 7.8, 1% Triton X-100, 15mM MgSO₄, 4mM EGTA, 1mM DTT) were pipetted into each well. After incubation for 5 minutes at room temperature the 96-well plate was analyzed in the Victor³ Multilabel Counter (PerkinElmer) with two-channel dispensing unit primed with the buffer solutions for firefly and, if necessary, *Renilla* luciferase activity measurements (prepared as described³⁹). The final volumes dispensed per well were 50µl of firefly and 50µl of *Renilla* solutions.

### Bacterial protein expression and purification

The pET-23b vector encoding 6xHis-tagged Wnt7a was prepared by cloning the sequence of the human Wnt7a in pOTB7 vector (Source Bioscience, clone IRAUp969F1132D) amplified using primers: forward: 5'-CTGTATACATATGCACCATCACCATCACCATGGCGCAAGCATCATC TGTAAC-3' and reverse 5'-GCAACTAGAAGGCACAGTCGAGG-3': using restriction sites NdeI and XhoI. The protein was subsequently expressed in Rosetta-gami (Merck-Millipore) *E.coli* strain by 4h induction with 1mM IPTG (CarlRoth) at 37°C, 150rpm. The cells were harvested and resuspended in 1X TBS (20mM Tris, 150mM NaCl) supplemented with 1mM PMSF (CarlRoth). After lysis in One-shot Cell Disruptor (Constant Systems) the lysate was separated by centrifugation for 15 min at 16000g, supernatant discarded and the pellet was resuspended in 1X TBS containing 1% Triton X-100. After 10 min on rotary shaker, the mixture was again separated for 15 min at 16000g, supernatant was discarded and the pellet was solubilized at 65°C in 1X TBS containing 8M urea and 1mM PMSF. Remaining insoluble matter was removed by centrifuging for 30 min at 16000g. Supernatant was then purified on Ni-NTA sepharose (Qiagen) in denaturing conditions (i.e. all the buffers contained 8M Urea) according to the manufacturer's protocol.

### Proteolytic digestion

For proteolytic digestion, the purified 6xHisWnt7a protein was precipitated from urea-containig buffer by adding 9 volumes of 100% ethanol. After incubation for 1h at 4°C the precipitate was pelleted for 30 min at 16000g, 4°C. The pellet was subsequently washed with 10 volumes of roomtemperature 20% ethanol and after centrifuging for 10 min at 16000g sheared to a fine powder in 1 volume of 20% ethanol. This mixture was supplemented with 9 volumes of 0.5% Trypsin-EDTA (Invitrogen) and incubated at 37°C on rotary shaker for 2-3h.

### Reverse-phase chromatography

The 1ml of mixture of tryptic peptides prepared from 3mg of 6xHisWnt7a protein was supplemented with 1% trifluoroacetic acid (TFA) and 2% acetonitrile. The mixture was centrifuged for 30min at 16000g. Supernatant was applied to a µRPC C2/C18 ST 4.6/100 column (Pharmacia) equilibrated with 0.1%TFA in 2% acetonitrile. After wash with 5 column volumes of binding buffer, the peptides were eluted with 2-step gradient of elution buffer (0.1% TFA, 80% acetonitrile). The obtained fractions were then supplemented with HCl to a final concentration of 20mM and evaporated in SpeedVac (Eppendorf) at 60°C. Dry residues were resuspended in 20µl of 1X PBS and diluted 1:10 in the culture medium supplemented with 200ng/ml Wnt3a for assay of the inhibitory activity. Peptide contents of active fractions was analysed with LC-MS at Unil Protein analysis facility. Candidate peptides were synthesized in Unil Protein and Peptide Chemistry facility with 70% purity. The stock was prepared in DMSO with a 100mg/ml final concentration for subsequent assays.

### Sequences

**Table 1. List of peptides of the invention**

| The peptides of the invention are preferably larger than 5aa | | | |
|---|---|---|---|
| | Positions | Amino acid sequence | SEQ ID NO: |
| 1 | 103-135 | EAAFTYAIIAAGVAHAITAA CTQGNLSDCGCDK | 1 |
| 2 | 50-78 | AICQSRPDAIIVIGEGSQMG LDECQFQFR | 2 |
| 3 | 274-292 | SPNYCEEDPVTGSVGTQGR | 3 |
| 4 | 23-40 | IGGFSSVVALGASIICNK | 4 |
| 5 | 8-22 | CLGHLFLSLGMVYLR | 5 |
| 6 | 297-311 | TAPQASGCDLMCCGR | 6 |
| 7 | 262-273 | KPMDTDLVYIEK | 7 |
| 8 | 178-189 | TLMNLHNNEAGR | 8 |
| 9 | 232-242 | YNEAVHVEPVR | 9 |
| 10 | 213-222 | TCWTTLPQFR | 10 |
| 11 | 312-320 | GYNTHQYAR | 11 |
| 12 | 328-335 | FHWCCYVK | 12 |
| 13 | 202-212 | CHGVSGSCTTK | 13 |
| 15 | 82-90 | WNCSALGER | 14 |
| 16 | 149-157 | WGGCSADIR | 15 |
| 17 | 321-327 | VWQCNCK | 16 |
| 18 | 191-197 | ILEENMK | 17 |
| 19 | 343-349 | TEMYTCK | 18 |
| 20 | 223-229 | ELGYVLK | 19 |
| 21 | 336-342 | CNTCSER | 20 |
| 22 | 138-143 | QGQYHR | 21 |
| 23 | 256-261 | KPLSYR | 22 |
| 24 | 248-253 | RPTFLK | 23 |
| 25 | 158-164 | YGIGFAK | 24 |
| 26 | 41-47 | IPGLAPR | 25 |
| 27 | 165-170 | VFVDAR | 26 |
| 28 | 144-148 | DEGWK | 27 |
| 29 | 91-95 | TVFGK | 28 |

### Reference List

1 Logan, C. Y. & Nusse, R. The Wnt signaling pathway in development and disease. Annu. Rev. Cell Dev. Biol. 20, 781-810 (2004).
2 Cliffe, A., Hamada, F. & Bienz, M. A role of Dishevelled in relocating Axin to the plasma membrane during wingless signaling. Curr. Biol. 13, 960-966 (2003).
3 Kalani, M. Y. et al. Wnt-mediated self-renewal of neural stem/progenitor cells. Proc. Natl. Acad. Sci. U. S. A. 105, 16970-16975 (2008).
4 Barker, N. & Clevers, H. Mining the Wnt pathway for cancer therapeutics. Nat. Rev. Drug Discov. 5, 997-1014 (2006).
5 Turashvili, G., Bouchal, J., Burkadze, G. & Kolar, Z. Wnt signaling pathway in mammary gland development and carcinogenesis. Pathobiology 73, 213-223 (2006).
6 Steinberg, I. Z. Long-range nonradiative transfer of electronic excitation energy in proteins and polypeptides. Annu. Rev. Biochem. 40, 83-114 (1971).
7 Dorsky, R. I., Sheldahl, L. C. & Moon, R. T. A transgenic Lef1/beta-catenin-dependent reporter is expressed in spatially restricted domains throughout zebrafish development. Dev. Biol. 241, 229-237 (2002).
8 Royer, C. A. Probing protein folding and conformational transitions with fluorescence. Chem. Rev. 106, 1769-1784 (2006).
9 Phillips, W. J. & Cerione, R. A. The intrinsic fluorescence of the alpha subunit of transducin. Measurement of receptor-dependent guanine nucleotide exchange. J. Biol. Chem. 263, 15498-15505 (1988).
10 Mixon, M. B. et al. Tertiary and quaternary structural changes in Gi alpha 1 induced by GTP hydrolysis. Science 270, 954-960 (1995).
11 Simons, M. & Mlodzik, M. Planar cell polarity signaling: from fly development to human disease. Annu. Rev. Genet. 42, 517-540 (2008).
12 Remmers, A. E. & Neubig, R. R. Partial G protein activation by fluorescent guanine nucleotide analogs. Evidence for a triphosphate-bound but inactive state. J. Biol. Chem. 271, 4791-4797 (1996).
13 Koval, A. & Katanaev, V. L. Platforms for High-1 Throughput Screening of Wnt/Frizzled Antagonists. Drug Discov. Today in press (2012).
14 Mikels, A. J. & Nusse, R. Purified Wnt5a Protein Activates or Inhibits β-Catenin- TCF Signaling Depending on Receptor Context. PLoS Biol 4, e115-e115 (2006).
15 Torres, M. A. et al. Activities of the Wnt-1 class of secreted signaling factors are antagonized by the Wnt-5A class and by a dominant negative cadherin in early Xenopus development. J. Cell Biol. 133, 1123-1137 (1996).
16 Kuhl, M. et al. Antagonistic regulation of convergent extension movements in Xenopus by Wnt/beta-catenin and Wnt/Ca2+ signaling. Mech. Dev. 106, 61-76 (2001).
17 Liang, H. et al. Wnt5a inhibits B cell proliferation and functions as a tumor suppressor in hematopoietic tissue. Cancer Cell 4, 349-360 (2003).
18 Pukrop, T. & Binder, C. The complex pathways of Wnt 5a in cancer progression. J Mol Med (Berl) 86, 259-266 (2008).
19 Jessen, J. R. Noncanonical Wnt signaling in tumor progression and metastasis. Zebrafish 6, 21-28 (2009).
20 Matsuda, Y., Schlange, T., Oakeley, E. J., Boulay, A. & Hynes, N. E. WNT signaling enhances breast cancer cell motility and blockade of the WNT pathway by sFRP1 suppresses MDA-MB-231 xenograft growth. Breast Cancer Res 11, R32 (2009).
21 Gurney, A. et al, Wnt pathway inhibition via the targeting of Frizzled receptors results in decreased growth and tumorigenicity of human tumors. Proc. Natl. Acad. Sci. U. S. A. (2012).
22 Yin, S., Xu, L., Bandyopadhyay, S., Sethi, S. & Reddy, K. B. Cisplatin and TRAIL enhance breast cancer stem cell death. Int. J. Oncol. 39, 891-898 (2011).
23 Benhaj, K., Akcali, K. C. & Ozturk, M. Redundant expression of canonical Wnt ligands in human breast cancer cell lines. Oncol. Rep. 15, 701-707 (2006).
24 Kim, M. et al. Functional interaction between Wnt3 and Frizzled-7 leads to activation of the Wnt/beta-catenin signaling pathway in hepatocellular carcinoma cells. J. Hepatol. 48, 780-791 (2008).
25 Katanaev, V. L. & Buestorf, S. Frizzled Proteins are bona fide G Protein-Coupled Receptors. Available from Nature Precedings <http://hdl.handle.net/10101/npre.2009.2765.1> (2009).
26 Le Grand, F., Jones, A. E., Seale, V., Scime, A. & Rudnicki, M. A. Wnt7a activates the planar cell polarity pathway to drive the symmetric expansion of satellite stem cells. Cell Stem Cell 4, 535-547 (2009).
27 Yang, L. et al. FZD7 has a critical role in cell proliferation in triple negative breast cancer. Oncogene (2011).
28 Yang, Y., Topol, L., Lee, H. & Wu, J. Wnt5a and Wnt5b exhibit distinct activities in coordinating chondrocyte proliferation and differentiation. Development 130, 1003-1015 (2003).
29 Howe, L. R. & Brown, A. M. Wnt signaling and breast cancer. Cancer Biol Ther 3, 36-41 (2004).
30 Safholm, A. et al. A formylated hexapeptide ligand mimics the ability of Wnt-5a to impair migration of human breast epithelial cells. J. Biol. Chem. 281, 2740-2749 (2006).
31 Khafizov, K. GoLoco motif proteins binding to Galpha(i1): insights from molecular simulations. J Mol Model 15, 1491-1499 (2009).
32 Hurley, J. B., Simon, M. I., Teplow, D. B., Robishaw, J. D. & Gilman, A. G. Homologies between signal transducing G proteins and ras gene products. Science 226, 860-862 (1984).
33 Mazzoni, M. R., Malinski, J. A. & Hamm, H. E. Structural analysis of rod GTP-binding protein, Gt. Limited proteolytic digestion pattern of Gt with four proteases defines monoclonal antibody epitope. J. Biol. Chem. 266, 14072-14081 (1991).
34 von Maltzahn, J., Zinoviev, R., Chang, N. C., Bentzinger, C. F. & Rudnicki, M. A. A truncated Wnt7a retains full biological activity in skeletal muscle. Nat Commun 4, 2869 (2013).
35 Stampfer, M. R. et al. Expression of the telomerase catalytic subunit, hTERT, induces resistance to transforming growth factor beta growth inhibition in p16INK4A(-) human mammary epithelial cells. Proc. Natl. Acad. Sci. U. S. A. 98, 4498-4503 (2001).
36 Sherman, M. Y., Meng, L., Stampfer, M., Gabai, V. L. & Yaglom, J. A. Oncogenes induce senescence with incomplete growth arrest and suppress the DNA damage response in immortalized cells. Aging Cell 10, 949-961 (2011).
37 Mosimann, C., Hausmann, G. & Basler, K. The role of Parafibromin/Hyrax as a nuclear Gli/Ci-interacting protein in Hedgehog target gene control. Mech. Dev. 126, 394-405 (2009).
38 Koval, A. & Katanaev, V. L. Wnt3a stimulation elicits G-protein-coupled receptor properties of mammalian Frizzled proteins. Biochem. J. 433, 435-440 (2011).
39 Dyer, B. W., Ferrer, F. A., Klinedinst, D. K. & Rodriguez, R. A noncommercial dual luciferase enzyme assay system for reporter gene analysis. Anal. Biochem. 282, 158-161 (2000).

## Claims

1. Polypeptide wherein the polypeptide is a fragment of a Wnt7a polypeptide, preferably it is a human sequence.

2. Polypeptide according to claim 1 wherein the polypeptide is **characterized by** a sequence according to Table 1.

3. Polypeptide according to claim 2 wherein the polypeptide is **characterized by** one or more modifications of one or more amino acids of the polypeptide.

4. Polypeptide according to claim 3 wherein the amino acid modification is a N-, C- or O-linked glycosylation, methylation, acylation, amidation, hydroxilation, or sulfation.

5. Composition comprising one or more polypeptides according to any of claims 1 to 4.

6. Composition according to claim 5 wherein at least two polypeptides are covalently linked to each other.

7. Polypeptide or composition according to any of claims 1 to 6 for medical use.

8. Polypeptide or composition according to any of claims 1 to 6 for use in preventing or treating a cancer.

9. Polypeptide or composition according to claim 6 wherein the cancer is a Wnt-dependent cancer or/and a triple-negative breast cancer.


SEQUENCE LISTING

<110>  University of Lausanne

<120>  Wnt7a polypeptides and their use

<130>  UL20246EP

<160>  28

<170>  PatentIn version 3.5

<210>  1
<211>  33
<212>  PRT
<213>  Homo sapiens

<400>  1

Glu Ala Ala Phe Thr Tyr Ala Ile Ile Ala Ala Gly Val Ala His Ala
1               5                   10                  15

Ile Thr Ala Ala Cys Thr Gln Gly Asn Leu Ser Asp Cys Gly Cys Asp
            20                  25                  30

Lys

<210>  2
<211>  29
<212>  PRT
<213>  Homo sapiens

<400>  2

Ala Ile Cys Gln Ser Arg Pro Asp Ala Ile Ile Val Ile Gly Glu Gly
1               5                   10                  15

Ser Gln Met Gly Leu Asp Glu Cys Gln Phe Gln Phe Arg
            20                  25

<210>  3
<211>  19
<212>  PRT
<213>  Homo sapiens

<400>  3

Ser Pro Asn Tyr Cys Glu Glu Asp Pro Val Thr Gly Ser Val Gly Thr
1               5                   10                  15

Gln Gly Arg

<210>  4
<211>  18

<212>  PRT
<213>  Homo sapiens

<400>  4

Ile Gly Gly Phe Ser Ser Val Val Ala Leu Gly Ala Ser Ile Ile Cys
1               5                   10                  15

Asn Lys

<210>  5
<211>  15
<212>  PRT
<213>  Homo sapiens

<400>  5

Cys Leu Gly His Leu Phe Leu Ser Leu Gly Met Val Tyr Leu Arg
1               5                   10                  15

<210>  6
<211>  15
<212>  PRT
<213>  Homo sapiens

<400>  6

Thr Ala Pro Gln Ala Ser Gly Cys Asp Leu Met Cys Cys Gly Arg
1               5                   10                  15

<210>  7
<211>  12
<212>  PRT
<213>  Homo sapiens

<400>  7

Lys Pro Met Asp Thr Asp Leu Val Tyr Ile Glu Lys
1               5                   10

<210>  8
<211>  12
<212>  PRT
<213>  Homo sapiens

<400>  8

Thr Leu Met Asn Leu His Asn Asn Glu Ala Gly Arg
1               5                   10

<210>  9
<211>  11
<212>  PRT
<213>  Homo sapiens

<400>  9

Tyr Asn Glu Ala Val His Val Glu Pro Val Arg
1               5                   10

<210>  10
<211>  10
<212>  PRT
<213>  Homo sapiens

<400>  10

Thr Cys Trp Thr Thr Leu Pro Gln Phe Arg
1               5                   10

<210>  11
<211>  9
<212>  PRT
<213>  Homo sapiens

<400>  11

Gly Tyr Asn Thr His Gln Tyr Ala Arg
1               5

<210>  12
<211>  8
<212>  PRT
<213>  Homo sapiens

<400>  12

Phe His Trp Cys Cys Tyr Val Lys
1               5

<210>  13
<211>  11
<212>  PRT
<213>  Homo sapiens

<400>  13

Cys His Gly Val Ser Gly Ser Cys Thr Thr Lys
1               5                   10

<210>  14
<211>  9
<212>  PRT
<213>  Homo sapiens

<400>  14

Trp Asn Cys Ser Ala Leu Gly Glu Arg
1               5

<210>  15
<211>  9
<212>  PRT

<213>  Homo sapiens

<400>  15

Trp Gly Gly Cys Ser Ala Asp Ile Arg
1               5


<210>  16
<211>  7
<212>  PRT
<213>  Homo sapiens

<400>  16

Val Trp Gln Cys Asn Cys Lys
1               5


<210>  17
<211>  7
<212>  PRT
<213>  Homo sapiens

<400>  17

Ile Leu Glu Glu Asn Met Lys
1               5


<210>  18
<211>  7
<212>  PRT
<213>  Homo sapiens

<400>  18

Thr Glu Met Tyr Thr Cys Lys
1               5


<210>  19
<211>  7
<212>  PRT
<213>  Homo sapiens

<400>  19

Glu Leu Gly Tyr Val Leu Lys
1               5


<210>  20
<211>  7
<212>  PRT
<213>  Homo sapiens

<400>  20

Cys Asn Thr Cys Ser Glu Arg
1               5

<210>  21
<211>  6
<212>  PRT
<213>  Homo sapiens

<400>  21

Gln Gly Gln Tyr His Arg
1               5


<210>  22
<211>  6
<212>  PRT
<213>  Homo sapiens

<400>  22

Lys Pro Leu Ser Tyr Arg
1               5


<210>  23
<211>  6
<212>  PRT
<213>  Homo sapiens

<400>  23

Arg Pro Thr Phe Leu Lys
1               5


<210>  24
<211>  7
<212>  PRT
<213>  Homo sapiens

<400>  24

Tyr Gly Ile Gly Phe Ala Lys
1               5


<210>  25
<211>  7
<212>  PRT
<213>  Homo sapiens

<400>  25

Ile Pro Gly Leu Ala Pro Arg
1               5


<210>  26
<211>  6
<212>  PRT
<213>  Homo sapiens

<400>  26

Val Phe Val Asp Ala Arg

1                   5

<210>  27
<211>  5
<212>  PRT
<213>  Homo sapiens

<400>  27

Asp Glu Gly Trp Lys
1               5

<210>  28
<211>  5
<212>  PRT
<213>  Homo sapiens

<400>  28

Thr Val Phe Gly Lys
1               5